Europäisches Patentamt

European Patent Office

Office européen des brevets

(19)

(11) Publication number: **0 178 682**

**A2**

(12) # EUROPEAN PATENT APPLICATION

(21) Application number: **85113260.5**

(22) Date of filing: **18.10.85**

(51) Int. Cl.⁴: **C 07 D 249/08**
**A 61 K 31/41**

(30) Priority: **19.10.84 US 662856**

(43) Date of publication of application:
**23.04.86 Bulletin 86/17**

(84) Designated Contracting States:
**AT BE CH DE FR GB IT LI LU NL SE**

(71) Applicant: **SCHERING CORPORATION**
**2000 Galloping Hill Road**
**Kenilworth, New Jersey 07033(US)**

(72) Inventor: **Desai, Jagdish Armitlal**
**114 Stuyvesant Avenue**
**Jersey City New Jersey 07306(US)**

(72) Inventor: **Copper, Alan Burce**
**1 Aldom Circle**
**West Caldwell New Jersey 07006(US)**

(74) Representative: **von Kreisler, Alek, Dipl.-Chem. et al,**
**Deichmannhaus am Hauptbahnhof**
**D-5000 Köln 1(DE)**

(54) **1,3-Bis(1H-1,2,4-triazol-1-yl)-2-fluoro-2-(2,4-difluorophenyl)propane, antifungal composition containing it, and process for preparation of the compound and the composition.**

(57) 1,3-Bis(1$H$-1,2,4-triazol-1-yl)-2-fluoro-2-(2,4-difluorophenyl)propane and its pharmaceutically acceptable salts and pharmaceutical composition containing same. The new compound is useful for treating fungal infection in animals, including humans.

EP 0 178 682 A2

TITLE MODIFIED
see front page

1,3-BIS(1H-1,2,4-TRIAZOL-1-YL)-2-FLUORO-2-(2,4-
DIFLUOROPHENYL)PROPANE, ANTIFUNGAL COMPOSITION
CONTAINING IT, AND PROCESS FOR PREPARATION OF THE
COMPOUND AND THE COMPOSITION

This invention relates to 1,3-bis(1H-1,2,4-
triazol-1-yl)-2-fluoro-2-(2,4-difluorophenyl)propane, an
antifungal compound, pharmaceutical compositions containing it and their use in treating fungal infections in
animals, including humans.

U.S. Patent 4,404,216 (Richardson) discloses
that 1,3-bis(1H-1,2,4-triazol-1-yl)-2-(2,4-difluorophenyl)-propan-2-ol and its pharmaceutically acceptable
acid addition salts are useful for treating fungal infections in animals.

European Patent Application No. 96,569, published December 21, 1983, and assigned to Pfizer Limited,
discloses 1,3-bis(1H-1,2,4-triazol-1-yl)-2-aryl-2-
halopropane antifungal compounds which are generic to the
compound of this invention. However, there is no
specific disclosure of the compound of this invention.

The compound of this invention, 1,3-bis-(1H-
1,2,4-triazol-1-yl)-2-fluoro-2-(2,4-difluorophenyl)propane, or a pharmaceutically acceptable salt thereof

possesses advantageous antifungal properties and low toxicity.

The present invention also provides pharmaceutical compositions comprising an effective antifungal amount of 1,3-bis(1H-1,2,4-triazol-1-yl)-2-fluoro-2-(2,4-difluorophenyl)propane or a pharmaceutically acceptable acid salt thereof, together with a pharmaceutically acceptable carrier or diluent. The pharmaceutical compositions of the present invention exhibit extended shelf-lifetime.

The present invention further provides a method of treating susceptible fungal infections which comprises administering to a host, e.g., a warm blooded animal such as man, in need of such treatment 1,3-bis-(1H-1,2,4-triazol-1-yl)-2-fluoro-2-(2,4-difluorophenyl)propane or a pharmaceutically acceptable salt thereof, or a pharmaceutical composition comprising such a compound or a salt thereof, together with a pharmaceutically acceptable carrier or diluent.

The compound of this invention may be prepared by reacting 1,3-bis(1H-1,2,4-triazol-1-yl)-2-(2,4-difluorophenyl)propan-2-ol with an active fluorinating reagent such as $SF_4$ or N,N-diethylaminosulfur trifluoride $(Et_2NSF_3)$ in a suitable organic solvent e.g., dry tetrahydrofuran (THF) or dry methylene chloride at a temperature of from about -10°C to the reflux temperature, preferably at about -10° to 0°C optionally in the presence of a base. The product is isolated and purified in a conventional manner, e.g. by column chromatography. 1,3-Bis(1H-1,2,4-triazol-1-yl)-2-(2,4-difluorophenyl)-propan-2-ol is a known compound which may be prepared in accordance with the procedures disclosed in USP 4,404,216.

The compound of the present invention and ketoconazole, a present commercial antifungal product, when tested in conventional antifungal screening tests, exhibited negligible in vitro activity against a large number of fungi in Sabourand broth medium but the compound of this invention exhibited significantly lower in vitro activity against six strains of Candida in Eagles medium compared to ketoconazole. The compound of this invention had a mean Minimum Inhibitory Concentration (MIC) of 4 mcg/mL after 48 hours; the MIC for ketoconazole was <0.031 mgc/mL. However, the compound of the present invention exhibited superior antifungal activity in in vivo tests in animals compared to ketoconazole. For example, in a mouse Candida albicans oral infection model, the preventive dosage of the compound of the present invention required for survival of 100% of the test animals was lower than that required for ketoconazole.

The present invention also provides a pharmaceutical composition comprising an effective antifungal amount of 1,3-bis(1H-1,2,4-triazol-1-yl)-2-fluoro-2-(2,4-difluorophenyl)propane or a pharmaceutically acceptable salt thereof and a pharmaceutically acceptable carrier or diluent.

The preferred pharmaceutically acceptable salts are nontoxic acid addition salts and/or adducts formed by adding to the compound of the present invention about a stoichiometric amount of a mineral acid, such as HCl, HBr, $H_2SO_4$ or $H_3PO_4$ or of an organic acid, such as acetic, propionic, valeric, oleic, palmitic, stearic, lauric, benzoic, lactic, para-toluene sulfonic, methane sulfonic, citric, maleic fumaric, succinic and the like, respectively.

The pharmaceutical compositions of the present invention may be formulated by combining the compound of this invention or a pharmaceutically acceptable salt and/or adduct thereof with any suitable, i.e., inert, pharmaceutical carrier or diluent adapted for administration orally, parentally or topically in a variety of
formulations. The preferred mode of administration is
oral.

Examples of suitable compositions include solid
or liquid compositions for oral administration such as
tablets, capsules, pills, powders, granules, solutions,
suspensions or emulsions. They may also be manufactured
in the form of sterile solid compositions which can be
dissolved in sterile water, physiological saline or some
other sterile injectable medium immediately before use.

Parenteral forms to be injected intravenously,
intramuscularly, or subcutaneously are usually in the
form of a sterile solution, and may contain salts or
glucose to make the solution isotonic.

Based on the greater in vivo oral activity for
the compound of this invention compared to ketoconazole,
the dosage of the compound of the present invention
employed to combat a given fungal infection in animals,
e.g., man, is likely to be generally somewhat less than
the dosage requirements of present commercial products
such as ketoconazole.

In general, the dosage for man ranges from
about 50 mg per day to about 800 per day, with about
100 mg to 400 mg per day in single or divided doses being
preferred.

It will be appreciated that the actual
preferred dosages of the compound of the present invention or pharmaceutically acceptable salts thereof will
vary according to the particular composition formulated,
the mode of application and the particular situs, host
and disease being treated. Many factors that modify the
action of the drug will be taken into account by the
attending clinician, e.g., age, body weight, sex, diet,
time of administration, rate of excretion, condition of the host, drug combinations, reaction sensitivities and severity of the disease. Administration can be carried out continuously or periodically within the maximum tolerated dose. Optimal application rates for a given set of conditions can be readily ascertained by the attending clinician using conventional dosage determination tests.

The following Examples illustrate the invention.

## EXAMPLE 1
### 1,3-Bis(1H-1,2,4-triazol-1-yl)-2-(2,4-difluorophenyl)-2-fluoro-propane

Dissolve 1,3-bis(1H-1,2,4-triazol-1-yl)-2-(2,4-difluorophenyl)propan-2-ol (4.76 g, 15.5 mmole) in 125 mL of dry THF under a nitrogen atmosphere. Cool to 0° to -10°C in a methanol/ice bath. Add, dropwise N,N-diethylaminosulfur trifluoride (5.00 g, 31.0 mmole) over a period of 10 minutes. Stir the reaction mixture so formed for 3 hours. Carefully pour the reaction mixture into 500 mL of aqueous saturated $NaHCO_3$ solution. Extract with two 500 mL portions of $CH_2Cl_2$. Dry the separated $CH_2Cl_2$ layers over $MgSO_4$, filter and evaporate to give a solid residue. Chromatograph the residue on a silica gel column eluting with 1:99, v/v methanol:chloroform to obtain the title compound:

1H NMR (90 MHz, $CDCl_3$) :δ8.09 (s,2H) 7.82 (s,2H), 7.4-6.6 (m,3H), 4.93 (dd,2H, J=18 and 15 Hz), 4.69 (s,2H)

Mass Spectrum, m/e (relative intensity): 55(88), 56(19), 82(72), 83(22), 119(27), 125(25), 127(96), 138(40), 139(38), 145(95), 152(65), 154(64), 158(98), 179(34), 181(100), 199(94), 219(90), 220(73), 226(65), 288(94), 289(20), and 308($M^+$,7).

EXAMPLE 2

The following are typical pharmaceutical formulations containing as the active ingredient (designated "Drug") the compound of this invention.

.

FORMULATION 1

| Tablet | 125.00 mg. tab. |
|---|---|
| Drug | 125.00 mg. |
| Polyethylene glycol 6000 | 100.00 mg. |
| Sodium lauryl sulfate | 6.25 mg. |
| Corn starch | 30.00 mg. |
| Lactose, anhydrous | 87.25 mg. |
| Magnesium stearate | 1.50 mg. |

Procedure:

Heat the polyethylene glycol 6000 to 70-80°C. Mix the drug, sodium lauryl sulfate, corn starch, and lactose into the liquid and allow the mixture to cool. Pass the solidified mixture through a mill. Blend granules with magnesium stearate and compress into tablets.

FORMULATION 2

| Capsule | 250 mg. tab. |
|---|---|
| Drug | 250.00 mg. |
| Lactose, anhydrous | 100.00 mg. |
| Corn starch | 50.00 mg. |
| Microcrystalline cellulose | 95.00 mg. |
| Magnesium stearate | 5.00 mg. |

Procedure:

0178682

Mix the first four ingredients in a suitable mixer for 10-15 minutes. Add the magnesium stearate and mix for 1-3 minutes. Fill the mixture into suitable two-piece hard gelatin capsules using an encapsulating machine.

0178682

CLAIMS:

1.      1,3-Bis(1H-1,2,4-triazol-1-yl)-2-fluoro-2-(2,4-difluorophenyl)propane or a pharmaceutically acceptable salt thereof.

2.      1,3-Bis(1H-1,2,4-triazol-1-yl)-2-fluoro-2-(2,4-difluorophenyl)propane or a pharmaceutically acceptable salt thereof as claimed in claim 1 for use in the treatment of fungal infections.

3.      A process for the preparation of 1,3-bis(1H-1,2,4-triazol-1-yl)-2-fluoro-2-(2,4-difluorophenyl)propane or pharmaceutically acceptable salts thereof as claimed in claim 1 or 2, characterized in that 1,3-bis(1H-1,2,4-triazol-1-yl)-2-(2,4-difluorophenyl)propan-2-ol is reacted with a fluorinating reagent followed by isolating the product in the free form or in the form of its pharmaceutically acceptable salt.

4.      Process according to claim 3, characterized in that $SF_4$ or N,N-diethylaminosulfur trifluoride is used as fluorinating agent.

5.      Pharmaceutical composition containing as active ingredient 1,3-bis(1H-1,2,4-triazol-1-yl)-2-fluoro-2-(2,4-difluorophenyl)propane or a pharmaceutically acceptable salt thereof together with a pharmaceutical carrier or excipient.

6.      Composition according to claim 5 in form suitable for oral administration.